# FASCICULE DE BREVET EUROPEEN

(11) **EP 3 749 400 B1**
(45) Date de publication et mention de la délivrance du brevet: **03.11.2021**
(21) Numéro de dépôt: 19711956.3
(22) Date de dépôt: 04.02.2019
(51) Int. Cl.: A61M 15/08, A61M 11/02, G16H 20/13, B05B 11/06

(54) **ENSEMBLE DE DISTRIBUTION DE PRODUIT FLUIDE**
ANORDNUNG FÜR DIE AUSGABE EINES FLÜSSIGPRODUKTS
ASSEMBLY FOR DISPENSING A FLUID PRODUCT

(30) Priorité: 07.02.2018 FR 1851001
(43) Date de publication de la demande: 16.12.2020
(73) Titulaire: APTAR France SAS, 27110 Le Neubourg (FR)
(72) Inventeur: NORRANT, Matthieu, 27400 Heudebouville (FR); HUPPÉ, Maxime, 76320 Caudebec Les Elbeuf (FR)
(74) Mandataire: CAPRI
(86) Numéro de dépôt international: PCT/FR2019/050239
(87) Numéro de publication internationale: WO 2019/155150

(56) Documents cités:
- WO-A1-02/085282
- WO-A1-2016/048435
- WO-A1-2016/075525
- WO-A1-2017/220879
- FR-A1- 3 024 655
- US-A1- 2013 063 579

## Description

La présente invention concerne un ensemble de distribution de produit fluide comportant un dispositif de distribution nasale de produit fluide et un appareil mobile distant, tel qu'un téléphone intelligent ou smartphone.

Les dispositifs de distribution nasale sont bien connus. Ils comportent généralement un réservoir contenant une ou plusieurs doses de produits fluide et une tête de distribution mobile par rapport audit réservoir pour distribuer le produit fluide, notamment via une pompe, une valve doseuse ou un piston coulissant dans ledit réservoir. Lorsque l'utilisateur souhaite utiliser le dispositif, il insère la tête de distribution dans la narine et actionne le dispositif pour distribuer une dose de produit fluide, généralement sous forme de spray.

Un inconvénient avec les dispositifs de l'art antérieur concerne l'efficacité de la dose distribuée dans la narine, en particulier lorsque le produit fluide distribué a pour objectif d'agir sur le cerveau. En effet, seule une partie minime de la dose atteint généralement la zone cible pour ce type de traitement, à savoir la zone olfactive comprenant les éthmoïdes, notamment en raison d'une orientation du dispositif d'administration dans la narine qui est variable d'un patient à un autre. Or, il apparait que cette orientation conditionne la bonne visée de la zone cible, en particulier dans le cas d'un spray fermé utilisé pour obtenir le maximum de déposition dans la zone cible.

Le document WO9857690 décrit un dispositif de distribution nasale comportant un moyen d'orientation en appui sur la lèvre supérieure de l'utilisateur. S'il améliore la qualité de l'insertion, un tel dispositif d'orientation ne permet pas de garantir une orientation optimale au moment de la distribution de la dose.

Les documents WO02085282 et FR3024655 décrivent d'autres dispositifs de l'état de la technique.

La présente invention a pour but de fournir un ensemble de distribution qui ne reproduit pas les inconvénients susmentionnés.

La présente invention a notamment pour but de fournir un ensemble de distribution nasale permettant de maîtriser l'orientation du dispositif dans la narine, quelle que soit la morphologie du patient et quelle que soit sa position, debout, couché ou incliné, au moment de l'utilisation du dispositif.

La présente invention a aussi pour but de fournir un ensemble de distribution nasale qui améliore le taux de déposition de produit actif sur la zone olfactive et/ou les éthmoïdes.

La présente invention a également pour but de fournir un ensemble de distribution nasale qui permette de renseigner l'utilisateur en temps réel sur la qualité d'insertion du dispositif dans la narine.

La présente invention a encore pour but de fournir un ensemble de distribution nasale qui permette à l'utilisateur de corriger l'orientation du dispositif dans la narine au moment de son actionnement.

La présente invention a également pour but de fournir un ensemble de distribution nasale qui est simple et peu coûteux à fabriquer et à assembler.

La présente a donc pour objet un ensemble de distribution de produit fluide, comportant :
- un dispositif de distribution nasale de produit fluide, ledit dispositif comportant un réservoir contenant du produit fluide, une tête de distribution étant assemblée sur ledit réservoir, ladite tête de distribution étant pourvue d'un orifice de distribution,
- un appareil mobile distant, tel qu'un smartphone,
dans lequel ledit dispositif de distribution comporte au moins un capteur d'orientation et communique avec ledit appareil mobile distant afin d'assister et guider l'utilisateur en temps réel pour obtenir une orientation optimale du dispositif de distribution au moment de l'actionnement,
dans lequel ledit capteur d'orientation, tel qu'un accéléromètre ou un gyroscope, détermine en temps réel l'orientation spatiale dudit dispositif, en particulier l'angle α dudit dispositif par rapport audit appareil mobile distant,
dans lequel ledit dispositif comporte un second capteur, tel qu'un accéléromètre, pour détecter quand le dispositif est actionné par l'utilisateur,
dans lequel ledit appareil mobile distant comporte un accéléromètre permettant de déterminer sa position dans l'espace, notamment l'angle β dudit appareil mobile distant par rapport à la verticale.

Avantageusement, ledit dispositif comporte un support supérieur et un support inférieur, disposés autour de ladite tête de distribution.

Avantageusement, ledit support supérieur est disposé au-dessus d'un repose-doigts de ladite tête de distribution, et ledit support inférieur est disposé autour d'une jupe de ladite tête de distribution.

Avantageusement, ledit dispositif comporte en outre un module de communication sans fil, avantageusement un module Bluetooth®, pour une communication avec ledit appareil mobile distant, et pour la retransmission en temps réel de la position dudit dispositif par rapport à la narine de l'utilisateur.

Avantageusement, ledit dispositif comporte un module électronique, tel qu'un circuit imprimé, comportant un microprocesseur contenant un logiciel de traitement des informations fournies par le ou les capteurs.

Avantageusement, ledit dispositif comporte au moins un dispositif d'indication visuel et/ou sonore et ou tactile, pour transmettre des informations en temps réel à l'utilisateur.

Avantageusement, ledit dispositif comprend un écran adapté à afficher des informations visibles par l'utilisateur

Avantageusement, ledit dispositif comprend un haut-parleur et/ou un élément vibrant, pour fournir une indication sonore et/ou tactile pour l'utilisateur.

Avantageusement, ledit appareil mobile distant comporte un écran et une caméra.

Avantageusement, ledit réservoir contient une seule dose ou seulement deux doses de produit fluide.

Ces caractéristiques et avantages et d'autres apparaîtront plus clairement au cours de la description détaillée suivante, faite en référence aux dessins joints, donnés à titre d'exemples non limitatifs, et sur lesquels :
- la figure 1 est une vue schématique en perspective d'un dispositif de distribution nasale selon un mode de réalisation avantageux,
- les figures 2 et 3 sont des vues schématiques en section transversale du dispositif de la figure 1, respectivement selon deux plans de coupe différents, en position de repos,
- les figures 4 à 6 sont des vues schématiques d'un utilisateur, respectivement en position debout, couchée et inclinée,
- la figure 7 est une vue schématique d'un écran, notamment de smartphone, en cours d'utilisation du dispositif par l'utilisateur, informant l'utilisateur en temps réel de l'orientation du dispositif dans la narine,
- la figure 8 est une vue similaire à celle de la figure 7, indiquant à l'utilisateur qu'il peut actionner le dispositif,
- les figures 9 et 10 sont des vues de détail représentant le dispositif tel qu'affiché sur l'écran de la figure 7, pour indiquer à l'utilisateur comment améliorer l'orientation du dispositif dans la narine avant l'actionnement, et
- les figures 11 à 13 sont des vue schématique en perspective du dispositif de la figure 1 après actionnement, avec un écran du dispositif affichant l'état de la distribution de dose, respectivement bonne, moyenne et mauvaise.

La présente invention concerne plus particulièrement un dispositif unidose, tel que divulgué dans le document WO0245866. Il est toutefois entendu que la présente invention ne se limite pas à ce type de dispositif, mais est au contraire applicable à tous les dispositifs de distribution de produit fluide et pulvérulents du type unidose, c'est-à-dire comportant un réservoir contenant une seule dose distribuée en un seul actionnement, bidose, c'est-à-dire comportant un réservoir contenant deux doses distribuées en deux actionnements successifs, ou multidoses, c'est-à-dire comportant un réservoir contenant plus de deux doses.

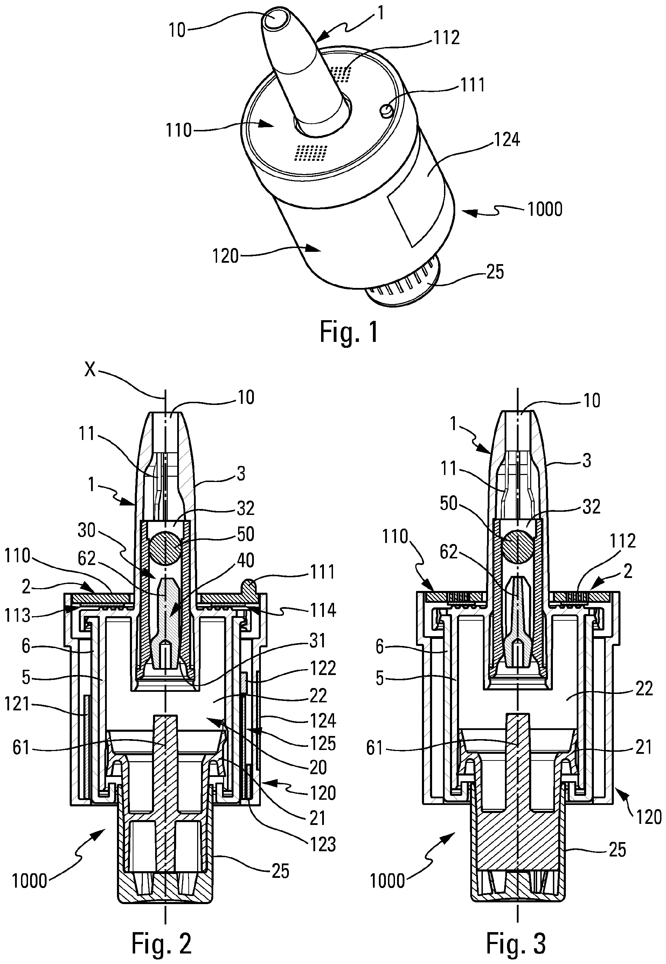

Dans la description ci-après, les termes "supérieur", "inférieur", "haut", "bas", "horizontal" et "vertical" se réfèrent à la position droite du dispositif représentée sur les figures 2, 3 et 11 à 13. Les termes "axial" et "radial" se réfèrent à l'axe central longitudinal X du dispositif, visible sur la figure 2.

Le dispositif 1000 donné à titre d'exemple est un unidose qui comporte un réservoir 30 comportant une entrée d'air 31 et une sortie de produit 32, et contenant une dose unique de produit fluide, liquide ou pulvérulent. L'entrée d'air 31 du réservoir est reliée à une chasse d'air 20 et la sortie de produit 32 du réservoir est reliée à un orifice de distribution 10 du dispositif. La sortie de produit 32 est obturée par un élément de fermeture 50 qui est emmanché à force dans ladite sortie de produit 32. L'entrée d'air 31 est pourvue d'un organe de retenue de produit 40 qui est adapté à maintenir le produit dans le réservoir 30 avant l'actionnement du dispositif. La chasse d'air 20 est actionnée manuellement par l'utilisateur et est adaptée à créer un écoulement d'air qui va traverser le réservoir 30 pour emmener le produit qu'il contient en direction de la sortie de distribution 10.

Le réservoir 30 est solidaire, notamment emmanché, dans une tête de distribution 1 qui comporte l'orifice de distribution 10. La tête de distribution 1 comporte avantageusement un repose-doigt 2 s'étendant radialement pour faciliter l'actionnement. Un manchon creux 3, formant embout nasal, s'étend axialement vers le haut à partir dudit repose-doigt 2 et se termine au niveau de ladite sortie de distribution 10. De préférence, ce manchon creux 3 est de dimension radiale réduite pour pouvoir être inséré dans une narine au moment de l'actionnement. Du côté opposé du repose-doigt 2, la tête de distribution 1 comporte une jupe 5 s'étendant axialement vers le bas à partir dudit repose-doigt 2. Eventuellement, un tube creux 6 peut être disposé radialement à l'extérieur de ladite jupe 5, comme visible sur les figures 2 et 3.

Le dispositif 1000 comporte un système d'ouverture mécanique 61, 62, qui est de préférence solidaire de la chasse d'air 20, c'est à dire qu'il est actionné simultanément à l'actionnement de ladite chasse d'air 20, et qui est adapté à coopérer avec ledit élément de fermeture 50 pour l'expulser mécaniquement de sa position d'obturation lors de l'actionnement du dispositif. Dans l'exemple représenté sur les figures, le système d'ouverture mécanique comporte un ensemble de tiges 61, 62, dont une première partie de tige 61 est solidaire de la chasse d'air 20, et une seconde partie de tige 62 est poussée par ladite première partie de tige 61 lorsque le dispositif est actionné. L'ensemble de tiges 61, 62 va en fin de course d'actionnement, c'est à dire en position de distribution, coopérer avec l'élément de fermeture 50 pour l'expulser mécaniquement de sa position d'obturation.

L'organe de retenue de produit 40 peut être avantageusement réalisé monobloc avec la seconde partie de tige 62. Ainsi, l'organe de retenue de produit 40 peut être réalisé de manière étanche au produit et étanche à l'air, avant l'actionnement du dispositif, la pression d'air créée par la chasse d'air 20 ne pénétrant à l'intérieur du réservoir 30 qu'au moment où ledit organe de retenu 40 est déplacé ensemble avec la seconde partie de tige 62, en étant poussé par la première partie de tige 61.

L'élément de fermeture 50 peut être sphérique, par exemple une bille, notamment en plastique, comme représenté sur les figures 2 et 3.

La chasse d'air 20, telle que représentée sur les figures 2 et 3, comporte un piston 21 coulissant dans une chambre d'air 22, le piston 21 étant actionné manuellement par l'utilisateur. La chambre d'air 22 peut être formée par ladite jupe 5 de la tête.

Avantageusement, l'actionnement du dispositif 1000 est réalisé au moyen d'un élément poussoir 25 assemblé sur ledit piston 21.

Le piston 21 est solidaire de la première partie de tige 61, avantageusement en étant formé sur une même pièce monobloc.

Lorsque l'utilisateur souhaite actionner le dispositif 1000, il place d'une part ses doigts sur le repose-doigt 2 de la tête de distribution 1 et d'autre part son pouce sur l'élément poussoir 25, et il exerce une force axiale d'actionnement, qui va déplacer la première partie de tige 61 et le piston 21 vers la position de distribution. Le piston 21 de la chasse d'air coopère de manière étanche avec la chambre d'air 22, de sorte que l'air contenu dans ladite chambre d'air 22 va être progressivement comprimé au cours de l'actionnement.

Après une course d'actionnement initiale de compression d'air, l'extrémité axiale supérieure de la première partie de tige 61 vient en contact de l'organe de retenue 40 et donc de la seconde partie de tige 62.

Une poursuite de l'actionnement déplace ledit organe de retenue 40 axialement vers le haut dans le réservoir 30, donc hors de sa position d'obturation ou de fermeture étanche de l'entrée d'air 32. A ce moment-là, l'air comprimé dans la chambre d'air 22 peut donc pénétrer dans le réservoir 30. Au même moment, l'extrémité axiale supérieure de la seconde partie de tige 62 vient en contact de l'élément de fermeture 50.

Une poursuite de l'actionnement va donc déplacer l'élément de fermeture 50 axialement vers le haut, hors de sa position d'obturation.

Lorsque l'étanchéité de l'élément de fermeture 50 est rompue, celui-ci est expulsé hors du réservoir 30 pour permettre la distribution du produit fluide ou pulvérulent sous l'effet de l'air comprimé. L'élément de fermeture 50 vient alors se coincer dans des nervures 11 de la tête de distribution 1, qui empêchent notamment tout risque d'expulsion dudit élément de fermeture 50 hors de ladite tête de distribution 1.

Bien entendu, la présente invention n'est pas limitée au dispositif unidose décrit ci-dessus, mais s'applique à tout dispositif nasal, qu'il soit unidose, bidose ou multidoses.

Selon l'invention, le dispositif nasal 1000 comporte au moins un capteur d'orientation et communique avec un appareil mobile distant 2000, notamment via une application dédiée, afin d'assister et guider l'utilisateur en temps réel pour obtenir une orientation optimale du dispositif de distribution au moment de son actionnement.

Un objectif est d'assurer un positionnement optimal du dispositif selon la morphologie du patient, et ce au moment même de l'actionnement du dispositif. Cela permet un meilleur taux de déposition du médicament dans la narine.

L'utilisateur peut éventuellement faire au préalable un test chez son spécialiste de santé afin de connaitre l'angle optimal adapté à sa morphologie, ce qui lui permet alors de configurer l'application associée.

De préférence, le dispositif 1000 comporte un support supérieur 110 et un support inférieur 120, disposés autour de la tête de distribution 1, lesdits supports supérieur et inférieur intégrant les éléments de l'invention. De cette manière, ni le fonctionnement, ni les performances du dispositif de distribution de produit fluide ne sont modifiés par la présente invention. En particulier, le support supérieur 110 peut être disposé au-dessus du repose-doigts 2 de la tête 1, et le support inférieur 120 peut être disposé autour de la jupe 5 de la tête 1 ou autour dudit tube creux 6.

Selon l'invention, le dispositif 1000 comporte un capteur d'orientation 113, tel qu'un accéléromètre ou un gyroscope, pour connaitre l'orientation spatiale du dispositif en temps réel, en particulier la position angulaire par rapport à l'appareil mobile distant.

Le dispositif 1000 comporte un second capteur 121, tel qu'un accéléromètre, pour détecter quand la dose est déclenchée par l'utilisateur. Ce second capteur peut être calibré précisément pour que seule l'accélération générée par l'actionnement du dispositif sera détectée.

L'appareil mobile distant 2000 comporte un accéléromètre permettant de déterminer sa position dans l'espace, notamment son angle β par rapport à la verticale.

Selon la position initiale du patient (debout, assis, incliné par exemple à ± 45° ou allongé), un logiciel recalcule les angles optimaux.

Le dispositif 1000 comporte en outre un module de communication sans fil 123, avantageusement un module Bluetooth®, pour une communication avec un appareil mobile distant 2000, tel qu'un smartphone ou une tablette, et pour la retransmission en direct de la position du dispositif par rapport à la narine de l'utilisateur.

Le dispositif 1000 comporte également un module électronique 125, tel qu'un circuit imprimé ou PCB, comportant un microprocesseur contenant le logiciel de traitement des informations fournies par le ou les capteurs.

Avantageusement, le dispositif 1000 comporte également au moins un dispositif d'indication visuel et/ou sonore et ou tactile, pour transmettre des informations à l'utilisateur. Ainsi, le dispositif peut comprendre un écran 124, adapté à afficher des informations visibles par l'utilisateur, et/ou une diode électroluminescente 111. Il peut aussi comprendre un haut-parleur 112 et/ou un élément vibrant 122, pour fournir une indication sonore et/ou tactile pour l'utilisateur, ce qui peut être utile pour des non-voyants par exemple.

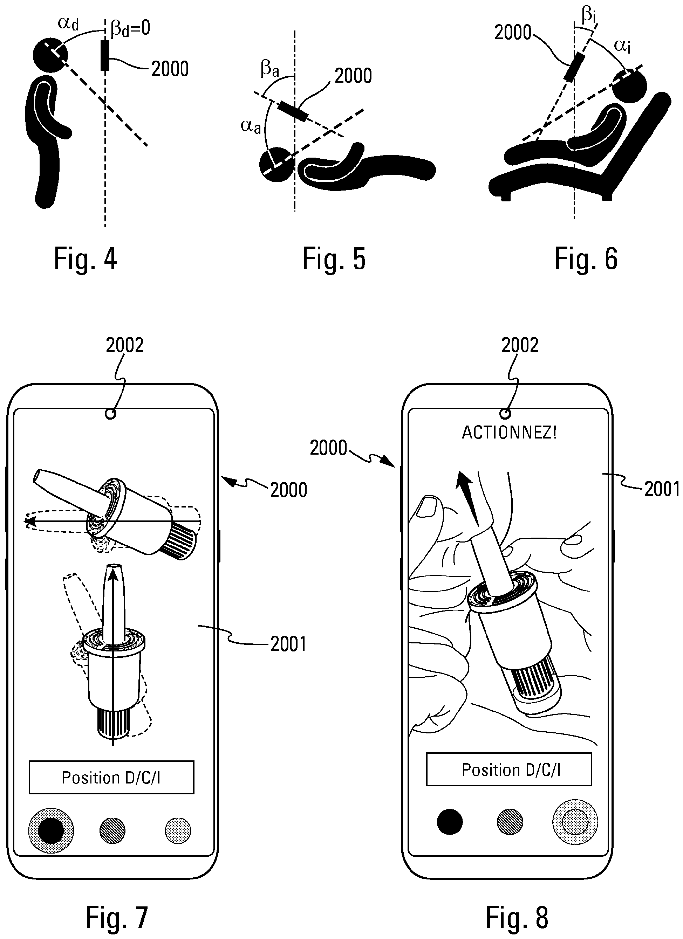

Le fonctionnement de l'ensemble va être décrit ci-après en référence aux figures 4 à 13. Dans cet exemple, l'appareil mobile distant 2000 est un smartphone.

Comme visible sur les figures 4 à 6, quelle que soit la position de l'utilisateur, debout (d) comme dans la figure 4, allongé (a) comme dans la figure 5 ou incliné (i) comme dans la figure 6, l'utilisateur place son visage en face de l'écran 2001 du smartphone, pour avoir le plan de l'écran parallèle au plan du visage. Avantageusement, on peut utiliser la caméra frontale 2002 du smartphone pour s'assurer que l'utilisateur regarde l'écran bien en face.

α_{d}, αₐ, αᵢ : angle du dispositif nasal par rapport au smartphone, toujours identique quelle que soit la position de l'utilisateur.

β_{d}, βₐ, βᵢ : angle du smartphone par rapport à la verticale, déterminé par l'accéléromètre du smartphone ; cette mesure permet de déterminer la position de l'utilisateur, et donc d'afficher le positionnement réel du dispositif sur l'écran du smartphone.

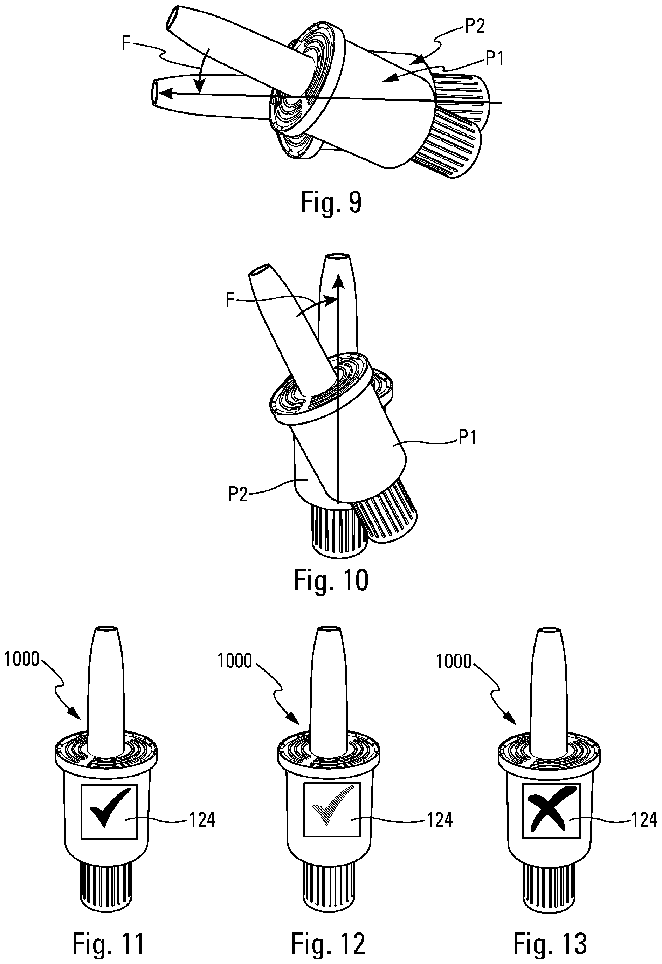

Description des étapes de fonctionnement :
- Position repos : l'accéléromètre du dispositif n'indique aucun mouvement ; l'électronique du dispositif est en veille ; l'application indique que le dispositif n'est pas dans la main du patient.
- Position première prise en main : l'accéléromètre du dispositif détecte le mouvement du dispositif ; l'électronique du dispositif est activée ; la combinaison des mesures d'angle α et β permet de déterminer la position de l'utilisateur et celle du dispositif par rapport audit utilisateur ; si le positionnement est hors de la zone optimale, comme illustré sur la figure 7, une indication, telle qu'une bulle rouge, s'affiche sur l'écran du smartphone ; parallèlement, l'écran affiche la position réelle P1 du dispositif dans l'espace ainsi que la position optimale P2, et une ou plusieurs flèches F peuvent guider l'utilisateur pour améliorer la position du dispositif vers la position optimale P2, comme illustré sur les figures 9 et 10 ; parallèlement, des indications sonores peuvent être émises, telles que par exemple « inclinez le dispositif vers le haut/bas » et/ou « tournez le dispositif vers votre gauche/droite » ; lorsque l'utilisateur s'approche de l'orientation optimale, une indication différente, telle qu'une bulle orange, peut s'afficher sur l'écran du smartphone.
- Position optimale : lorsque l'utilisateur à positionné le dispositif dans la position optimale, comme illustré sur la figure 8, une indication différente, telle qu'une bulle verte, peut s'afficher sur l'écran du smartphone, et l'utilisateur peut actionner le dispositif pour distribuer la dose ; parallèlement, l'écran du smartphone peut afficher un message incitant l'utilisateur à actionner le dispositif, tel que par exemple le mot « actionnez » et/ou une flèche animée indiquant qu'il faut déclencher la dose ; de plus, une indication sonore peut être émise, telle que par exemple « actionnez le dispositif » et/ou « déclenchez la dose ».
- Si lors de l'actionnement le dispositif s'est écarté de la zone optimale, des messages visuels ou sonores peuvent être émis, tels que par exemple « dose non optimale » ou « risque de dose insuffisante ».
- Après actionnement, l'écran 2001 du smartphone 2000, et éventuellement aussi l'écran 124 du dispositif 1000, s'il est prévu, peuvent afficher l'état de la dose qui a été émise, en fonction de la position du dispositif au moment précis de la distribution, tel que par exemple « Dose correcte », « Dose non optimale » ou « Risque de dose insuffisante ». Les figures 11 à 13 illustrent des affichages possibles sur l'écran 124 du dispositif 1000.

La présente invention procure ainsi de nombreux avantages :
- elle favorise un positionnement angulaire précis au moment de la distribution de dose, assurant ainsi un taux de déposition optimal du produit distribué dans la narine ;
- elle fournit une assistance « en temps réel » pour l'orientation, avec notamment la sécurité d'une validation de la position optimale avant l'actionnement ;
- elle fournit une solution utilisable avec un grand nombre de dispositifs de distribution nasale du même type, et n'est donc pas limitée à l'exemple décrit ;
- elle a un impact mineur sur la conception et l'encombrement du dispositif, et reste de ce fait facilement transportable ;
- elle n'a pas d'impact sur le fonctionnement et les performances du dispositif, et ne modifie donc pas les performances du produit fluide distribué ;
- elle permet d'administrer facilement le produit fluide à un tiers ;
- elle permet l'utilisation en toute position, notamment debout, assis, allongé ;
- elle permet un transfert de données aux médecins, spécialistes de santé, pharmaciens, réglementaire, assurances ;
- elle génère un auto-apprentissage : trouver l'angle optimal deviendra de plus en plus facile pour l'utilisateur, qui mémorisera inconsciemment la bonne position du dispositif par rapport à sa morphologie.

La présente invention a été décrite en référence à plusieurs modes de réalisation, mais il est entendu qu'un homme du métier peut y apporter toutes modifications, sans sortir du cadre de la présente invention tel que défini par les revendications annexées.

## Revendications

1. Ensemble de distribution de produit fluide, comportant:
- un dispositif de distribution nasale de produit fluide (1000), ledit dispositif comportant un réservoir (30) contenant du produit fluide, une tête de distribution (1) étant assemblée sur ledit réservoir (30), ladite tête de distribution (1) étant pourvue d'un orifice de distribution (10),
- un appareil mobile distant (2000), tel qu'un smartphone,
dans lequel ledit dispositif de distribution (1000) comporte au moins un capteur d'orientation (113) et communique avec ledit appareil mobile distant (2000) afin d'assister et guider l'utilisateur en temps réel pour obtenir une orientation optimale du dispositif de distribution (1000) au moment de l'actionnement,
dans lequel ledit capteur d'orientation (113), tel qu'un accéléromètre ou un gyroscope, détermine en temps réel l'orientation spatiale dudit dispositif (1000), en particulier l'angle (a) dudit dispositif (1000) par rapport audit appareil mobile distant (2000),
**caractérisé en ce que** ledit dispositif (1000) comporte un second capteur (121), tel qu'un accéléromètre, pour détecter quand le dispositif (1000) est actionné par l'utilisateur,
et **en ce que** ledit appareil mobile distant (2000) comporte un accéléromètre permettant de déterminer sa position dans l'espace, notamment l'angle (β) dudit appareil mobile distant (2000) par rapport à la verticale.

2. Ensemble selon la revendication 1, dans lequel ledit dispositif (1000) comporte un support supérieur (110) et un support inférieur (120), disposés autour de ladite tête de distribution (1).

3. Ensemble selon la revendication 2, dans lequel ledit support supérieur (110) est disposé au-dessus d'un repose-doigts (2) de ladite tête de distribution (1), et ledit support inférieur (120) est disposé autour d'une jupe (5) de ladite tête de distribution (1).

4. Ensemble selon l'une quelconque des revendications précédentes, dans lequel ledit dispositif (1000) comporte en outre un module de communication sans fil (123), avantageusement un module Bluetooth®, pour une communication avec ledit appareil mobile distant (2000), et pour la retransmission en temps réel de la position dudit dispositif (1000) par rapport à la narine de l'utilisateur.

5. Ensemble selon l'une quelconque des revendications précédentes, dans lequel ledit dispositif (1000) comporte un module électronique (125), tel qu'un circuit imprimé, comportant un microprocesseur contenant un logiciel de traitement des informations fournies par le ou les capteurs (113, 121).

6. Ensemble selon l'une quelconque des revendications précédentes, dans lequel ledit dispositif (1000) comporte au moins un dispositif d'indication visuel et/ou sonore et ou tactile, pour transmettre des informations en temps réel à l'utilisateur.

7. Ensemble selon la revendication 6, dans lequel ledit dispositif (1000) comprend un écran (124) adapté à afficher des informations visibles par l'utilisateur

8. Ensemble selon la revendication 6 ou 7, dans lequel ledit dispositif (1000)comprend un haut-parleur (112) et/ou un élément vibrant (122), pour fournir une indication sonore et/ou tactile pour l'utilisateur.

9. Ensemble selon l'une quelconque des revendications précédentes, dans lequel ledit appareil mobile distant (2000) comporte un écran (2001) et une caméra (2002).

10. Ensemble selon l'une quelconque des revendications précédentes, dans lequel ledit réservoir (30) contient une seule dose ou seulement deux doses de produit fluide.

## Patentansprüche

1. Fluidprodukt-Verteilungsanordnung, die Folgendes umfasst:
- eine Vorrichtung (1000) zur nasalen Fluidproduktverteilung, wobei die Vorrichtung einen Vorratsbehälter (30), der das Fluidprodukt enthält, und einen Verteilungskopf (1), der an den Vorratsbehälter (30) angefügt ist, umfasst, wobei der Verteilungskopf (1) mit einer Verteilungsöffnung (10) versehen ist,
- ein entferntes mobiles Gerät (2000) wie etwa ein Smartphone,
wobei die Verteilungsvorrichtung (1000) wenigstens einen Orientierungssensor (113) enthält und mit dem entfernten mobilen Gerät (2000) kommuniziert, um den Anwender in Echtzeit zu unterstützen und anzuleiten, um eine optimale Orientierung der Verteilungsvorrichtung (1000) zum Zeitpunkt der Betätigung zu erzielen,
wobei der Orientierungssensor (113) wie etwa ein Beschleunigungsmesser oder ein Gyroskop in Echtzeit die räumliche Orientierung der Vorrichtung (1000), insbesondere den Winkel (α) der Vorrichtung (1000) in Bezug auf das entfernte mobile Gerät (2000) bestimmt,
**dadurch gekennzeichnet, dass**
die Vorrichtung (1000) einen zweiten Sensor (121) wie etwa einen Beschleunigungsmesser enthält, um zu detektieren, wann die Vorrichtung (1000) durch den Anwender betätigt wird, und
dass das entfernte mobile Gerät (2000) einen Beschleunigungsmesser enthält, der ermöglicht, seine Position im Raum, insbesondere den Winkel (β) des entfernten mobilen Geräts (2000) in Bezug auf die Vertikale zu bestimmen.

2. Anordnung nach Anspruch 1, wobei die Vorrichtung (1000) einen oberen Träger (110) und einen unteren Träger (120), die um den Verteilungskopf (1) angeordnet sind, umfasst.

3. Anordnung nach Anspruch 2, wobei sich der obere Träger (110) über einer Fingerauflage (2) des Verteilungskopfes (1) befindet und der untere Träger (120) um eine Schürze (5) des Verteilungskopfes (1) angeordnet ist.

4. Anordnung nach einem der vorhergehenden Ansprüche, wobei die Vorrichtung (1000) außerdem ein Modul (123) für drahtlose Kommunikation, vorteilhaft ein Bluetooth^{®}-Modul, für die Kommunikation mit dem entfernten mobilen Gerät (2000) und für die Rücksendung in Echtzeit der Position der Vorrichtung (1000) in Bezug auf das Nasenloch des Anwenders umfasst.

5. Anordnung nach einem der vorhergehenden Ansprüche, wobei die Vorrichtung (1000) ein elektronisches Modul (125) wie etwa eine gedruckte Schaltung umfasst, die einen Mikroprozessor aufweist, der Software für die Verarbeitung der Informationen, die von dem einen oder den mehreren Sensoren (113, 121) geliefert werden, enthält.

6. Anordnung nach einem der vorhergehenden Ansprüche, wobei die Vorrichtung (1000) wenigstens eine Vorrichtung für die visuelle und/oder akustische und/oder taktile Angabe umfasst, um Informationen in Echtzeit an den Anwender zu übertragen.

7. Anordnung nach Anspruch 6, wobei die Vorrichtung (1000) einen Bildschirm (124) umfasst, der dafür ausgelegt ist, für den Anwender sichtbare Informationen anzuzeigen.

8. Anordnung nach Anspruch 6 oder 7, wobei die Vorrichtung (1000) einen Lautsprecher (112) und/oder ein Vibrationselement (122) umfasst, um eine akustische und/oder taktile Angabe für den Anwender zu liefern.

9. Anordnung nach einem der vorhergehenden Ansprüche, wobei das entfernte mobile Gerät (2000) einen Bildschirm (2001) und eine Kamera (2002) enthält.

10. Anordnung nach einem der vorhergehenden Ansprüche, wobei der Vorratsbehälter (30) eine einzige Dosis oder lediglich zwei Dosen des Fluidprodukts enthält.

## Claims

1. A fluid dispenser assembly, comprising:
· a nasal fluid dispenser device (1000), said device comprising a reservoir (30) containing fluid, a dispenser head (1) being assembled on said reservoir (30), said dispenser head (1) being provided with a dispenser orifice (10),
· a remote mobile device (2000), such as a smartphone,
wherein said dispenser device (1000) includes at least one orientation sensor (113) and communicates with said remote mobile device (2000) in order to assist and guide the user in real time, so as to obtain optimal orientation of the dispenser device (1000) when said device is actuated,
wherein said orientation sensor (113), such as an accelerometer or a gyro, determines in real time the three-dimensional orientation of said device (1000), in particular the angle (α) of said device (1000) relative to said remote mobile device (2000),
**characterized in that** said device (1000) includes a second sensor (121), such as an accelerometer, for detecting when the device (1000) is actuated by the user,
and **in that** said remote mobile device (2000) includes an accelerometer that makes it possible to determine its position in three-dimensional space, in particular the angle (β) of said remote mobile device (2000) relative to the vertical.

2. An assembly according to claim 1, wherein said device (1000) includes a top support (110) and a bottom support (120) that are arranged around said dispenser head (1).

3. An assembly according to claim 2, wherein said top support (110) is arranged above a finger rest (2) of said
dispenser head (1), and said bottom support (120) is arranged around a skirt (5) of said dispenser head (1).

4. An assembly according to any preceding claim, wherein said device (1000) further includes a wireless communication module (123), advantageously a Bluetooth® module, for communicating with said remote mobile device (2000), and for relaying in real time the position of said device (1000) relative to the user's nostril.

5. An assembly according to any preceding claim, wherein said device (1000) includes an electronic module (125), such as a printed circuit, including a microprocessor containing software for processing information provided by the sensor(s) (113, 121).

6. An assembly according to any preceding claim, wherein said device (1000) includes at least one visual and/or audible and/or tactile indicator device for transmitting information in real time to the user.

7. An assembly according to claim 6, wherein said device (1000) includes a screen (124) that is adapted to display information that can be seen by the user.

8. An assembly according to claim 6 or claim 7, wherein said device (1000) includes a loudspeaker (112) and/or a vibrator element (122) for providing the user with audible and/or tactile information.

9. An assembly according to any preceding claim, wherein said remote mobile device (2000) includes a screen (2001) and a camera (2002).

10. An assembly according to any preceding claim, wherein said reservoir (30) contains a single dose or only two doses of fluid.
